# EUROPEAN PATENT SPECIFICATION

(11) **EP 4 442 302 B1**
(45) Date of publication and mention of the grant of the patent: **07.10.2026**
(21) Application number: 22880444.9
(22) Date of filing: 07.11.2022
(51) Int. Cl.: A61M 15/00

(54) **NEBULIZER CUP AND USE THEREOF IN PREPARATION OF A DEVICE FOR NEBULIZED INHALATION ADMINISTRATION**
VERNEBLERBECHER UND VERWENDUNG DAVON BEI DER HERSTELLUNG EINER VORRICHTUNG ZUR VERNEBLERINHALATION
COUPELLE DE NÉBULISEUR ET SON UTILISATION DANS LA FABRICATION D'UN DISPOSITIF D'ADMINISTRATION PAR INHALATION PAR NÉBULISATION

(30) Priority: 29.11.2021 CN 202111429885
(43) Date of publication of application: 09.10.2024
(73) Proprietor: Cansino Biologics Inc., Tianjin 300457 (CN); Stamford Devices Limited, Dangan H91 EH6C Galway (IE)
(72) Inventor: SI, Weixue, Tianjin 300457 (CN); ZHAO, Xiaolong, Tianjin 300457 (CN); WANG, Yueran, Tianjin 300457 (CN); JIANG, Yueying, Tianjin 300457 (CN); WANG, Liping, Tianjin 300457 (CN); YU, Xuefeng, Tianjin 300457 (CN); CHAO, Shoubai, Tianjin 300457 (CN); ZHU, Tao, Tianjin 300457 (CN)
(74) Representative: HGF
(86) International application number: PCT/CN2022/130338
(87) International publication number: WO 2023/061508

(56) References cited:
- WO-A1-2011/129787
- CN-A- 105 916 491
- CN-A- 110 013 588
- CN-A- 110 013 588
- CN-A- 111 297 835
- CN-U- 203 829 437
- CN-U- 213 789 374
- GB-A- 2 494 304
- US-A- 5 596 982
- US-A1- 2003 205 226
- US-A1- 2004 050 966
- US-A1- 2008 210 225
- US-A1- 2008 257 345
- US-A1- 2013 276 781
- US-A1- 2016 237 288
- US-A1- 2019 125 991

## Description

### Technical Field

The present invention relates to the field of biomedicine, and in particular, to a nebulization cup and application thereof in preparation of a device for nebulized inhalation administration, especially for nebulized inhalation administration of a preventive and/or therapeutic drug for a respiratory disease (such as SARS-CoV-2 vaccine).

### Background Art

At present, one of the treatments for respiratory diseases is nebulized inhalation administration, which disperses the liquid drug into fine mist droplets by using a nebulizer and inhales them through a patient's nose or mouth. The nebulized drug directly acts on the target organs, which can increase the local concentration of the drug to achieve the therapeutic effect. Nebulized inhalation has a very good therapeutic effect on bronchiolitis, asthmatic bronchitis, bronchial asthma, acute and chronic bronchitis, acute laryngitis and acute pneumonia and the like.

US2013276781A1 discloses a nebulization cup, wherein the nebulization cup comprises a cup body and a cup lid, a material of the cup body is transparent plastic, and an antistatic agent is added to the cup body. US2019125991A1 discloses a transparent spacer with a body and a lid, wherein the body comprises an antistatic agent. US2008257345A1 discloses a transparent spacer with a body and a lid, wherein the body comprises an antistatic agent.

Nebulized inhalation vaccine is a vaccine that achieves immunization by nebulized inhalation. The so-called nebulized inhalation immunization means that the vaccine is nebulized into tiny particles through a nebulizer, and these tiny particles enter the respiratory tract and lungs through breathing inhalation to stimulate mucosal immunization.

The equipment and devices for implementing the nebulized inhalation therapy and immunization are very important for the implementation and quality of therapy and immunization, and have attracted great attention from researchers. The existing nebulized administration devices in the prior art mainly use the mode of nebulizing while inhaling for administration, and it is necessary to guide the subject before administration. For children and even many adults, the inhalation and absorption of drug mist is often greatly affected due to improper control of the frequency of inhalation administration and self-breathing, which is very unfavorable for immunization. In addition, for immunization, in order to prevent cross-infection, the part of the device for inhalation by the subject should be disinfected or discarded after use. In view of the necessity of large-scale inoculation, it is an urgent problem to provide a nebulized administration device which can prevent cross-infection, reduce cost with and achieve high inhalation inoculation efficiency.

Additionally, when using a nebulization cup for nebulized inhalation, the atomized medication mist tends to liquefy and form droplets on the walls of the cup due to static electricity, which cannot be normally inhaled by the human body. Therefore, it is necessary to add an antistatic agent to the nebulization cup to resist static electricity and prevent the liquefaction and residue of the medication mist. The dosage of the antistatic agent also has a significant impact on the medication mist. When the content of the antistatic agent is low, there is more deposition of the mist and a larger residue; when the content of the antistatic agent is high, it can affect the molding of the cup, the tactile sensation, and pose a threat to the effective amount of the atomized medication mist. Therefore, it is urgent to find the appropriate antistatic agent and the optimal dosage of the antistatic agent to ensure the effectiveness of the medication atomization.

### Summary of the Invention

Features of the invention are set forth in the appended claims.

In order to overcome the shortcomings of the prior art, the present invention provides a nebulization cup and application thereof. The nebulization cup can be used for collecting and carrying the drug mist generated by the nebulizer, and providing the drug mist be inhaled by the subject to achieve the effects of treatment and immunization.

In a first aspect of the present disclosure, a nebulization cup is provided, the nebulization cup includes a cup body and a cup lid, and an antistatic agent is added to the cup body.

Specifically, an antistatic agent is further added to the cup lid.

Specifically, the antistatic agent may be an anionic antistatic agent (such as alkyl sulfonate, alkyl phosphate, copolymer salt of maleic anhydride and other monomer copolymer salts, polyacrylate, polystyrene sulfonate), a zwitterionic antistatic agent (such as amphoteric alkyl imidazoline salt, alkyl amino acid), a nonionic antistatic agent (such as polyhydroxy alcohol fatty acid ester (such as glycerol fatty acid ester, sorbitan fatty acid ester)), polyethylene oxide additive (such as polyoxyethylene sorbitan fatty acid ester, polyoxyethylene fatty ether, polyoxyethylene alkylbenzene ether, polyoxyethylene alkylamine and the like), a polymer antistatic agent (such as polyoxyethylene fatty ether, polyoxyethylene alkylbenzene ether, polyethylene glycol fatty acid ester, polyacrylic acid derivative), or a composite antistatic agent and the like, especially a nonionic antistatic agent or a polymer antistatic agent.

Further, the nonionic antistatic agent mentioned is preferably selected from one of or a combination of more than one of fatty alcohol ethoxylates, alkyl phenol ethoxylates, and mono- and di-glycerides of fatty acids; the amphoteric antistatic agent is alkyl dicarboxymethyl ammonium succinimide and/or dodecyl dimethyl betaine; the polymer antistatic agent is selected from one of or a combination of more than one of ethylene oxide-propylene oxide adducts of ethylenediamine, poly(4-vinylpyridine) type polymeric soaps, octylphenyl styrenes, and styrene sulfonate copolymeric soaps.

Cationic quaternary ammonium salts have strong adhesion to polymer materials and good antistatic properties, and are also a kind of antistatic agents that are widely used in plastics. But they are irritating to skin and are toxic, so they do not belong to the selection range of the present disclosure.

In some embodiments of the present disclosure, an antistatic agent is added to the cup, the content of the antistatic agent is above 0.03%(w/w), especially above 0.05%, such as 0.03%-10% (such as 0.05%, 0.1%, 0.2%, 0.25%, 0.5%, 1%, 1.5%, 2%, 3%, 4%, 5%, 6%, 7%, 8%, 9%, 10%), especially 0.05%-5%.

In some embodiments of the present disclosure, an antistatic agent is added to the cup lid, the content of the antistatic agent is more than 0.03%(w/w), especially more than 0.05%, such as 0.03%-10% (such as 0.05%, 0.1%, 0.2%, 0.25%, 0.5%, 1%, 1.5%, 2%, 3%, 4%, 5%, 6%, 7%, 8%, 9%, 10%), especially 0.05%-5%.

In some embodiments of the present disclosure, an antistatic agent is added to both the cup body and the cup lid.

Specifically, the addition of the antistatic agent in the cup body and/or the cup lid may be achieved by adding the antistatic agent raw materials to its preparation raw materials, or by adding antistatic masterbatch to its preparation raw materials, especially by adding antistatic masterbatch to its preparation raw materials. The antistatic agent and the antistatic masterbatch may be selected from suitable commercial products according to the plastic material of the cup body and/or the cup lid.

Further, the cup lid is provided with a mist inlet and a suction nozzle.

Specifically, the mist inlet may be provided at any suitable position of the cup lid, such as the edge, the center, especially the edge of the cup lid; the mist inlet may also be provided with a closing member to facilitate closing the mist inlet before the nebulization cup is used.

Specifically, the suction nozzle is protrusively provided on the cup lid and communicated with the inner space of the cup. The suction nozzle may take any shape suitable for the subject to suck the mist, such as a round or conical shape, and the suction port end of the suction nozzle may take a shape suitable for the subject to suck, such as a round or oval shape. The suction port of the suction nozzle may also be provided with a closing member to facilitate closing the suction nozzle before the nebulization cup is used.

Specifically, the material of the cup lid may be a plastic, especially a medical grade plastic, such as polypropylene (PP), polyethylene (PE), polyvinyl chloride (PVC), polystyrene (PS), polycarbonate (PC), polylactic acid (PLA) and the like, especially PP and PLA. Specifically, the cup lid is transparent.

Specifically, the material of the cup body may be a plastic, especially a medical grade plastic, such as polypropylene (PP), polyethylene (PE), polyvinyl chloride (PVC), polystyrene (PS), polycarbonate (PC), polylactic acid (PLA) and the like, especially PP and PLA. Specifically, the cup body is transparent.

Specifically, the cup body and the cup lid may be integrally formed, or may be detachably provided, for example, connected by a screw thread or a buckle.

Specifically, the cup body may have any suitable shape, such as a cylindrical shape, a truncated cone shape with a tapered upper width and a lower width, and the like.

Specifically, the side wall of the cup body may also be provided with a handle to facilitate taking the nebulization cup.

Specifically, the volume of the nebulization cup may be 300 ml-800 ml (such as 300 ml, 350 ml, 400 ml, 450 ml, 500 ml, 550 ml, 600 ml, 700 ml, and 800 ml), especially 500 ml.

In a second aspect of the present disclosure, a method for preparing the nebulization cup described in the first aspect is provided. The method includes a step of adding an antistatic agent component or an antistatic masterbatch to the raw material for preparing the cup body.

In some embodiments of the present disclosure, the method further includes a step of adding an antistatic agent component or antistatic masterbatch to the raw material for preparing the cup lid.

Specifically, the method further includes a forming step, such as forming by extrusion, injection molding, compression molding, blow molding or other methods.

In a third aspect of the present disclosure, an application of the nebulization cup described in the first aspect in the preparation of a nebulized inhalation administration device is provided.

Specifically, the application is application of the nebulization cup in the preparation of a device for nebulized inhalation administration of a preventive and/or therapeutic drug for a respiratory disease.

Specifically, the device includes the nebulization cup described in the first aspect of the present disclosure, and a nebulizer; more specifically, the nebulizer may be an ultrasonic nebulizer, a compression nebulizer, a vibrating mesh screen nebulizer or other suitable nebulizers, especially a vibrating mesh screen nebulizer.

Specifically, the drug is a vaccine, such as pneumococcal vaccine, influenza vaccine, coronavirus vaccine, varicella vaccine, Newcastle virus vaccine, measles vaccine, tuberculosis vaccine, dust mite allergy vaccine, and the like.

In some embodiments of the present disclosure, the coronavirus vaccine is SARS-CoV-2 vaccine.

Specifically, the SARS-CoV-2 vaccine may be a recombinant adenovirus vector vaccine in which the S protein gene of SARS-CoV-2 is inserted into the recombinant adenovirus. More specifically, the recombinant adenovirus may also contain genes (full-length or partial sequences) of other structural proteins (such as M protein, E protein and N protein) of SARS-CoV-2.

Specifically, the above-mentioned adenovirus may be a human adenovirus (such as AdHu2 type, AdHu5 type and the like), an animal adenovirus vector such as a chimpanzee adenovirus vector (such as AdC6 type, AdC7 type, AdC36 type, AdC68 type and the like). In some embodiments of the present disclosure, the above-mentioned adenovirus is AdHu5.

Specifically, the content of recombinant adenovirus in the above-mentioned recombinant adenovirus vector vaccine is 1×10⁹ VP/ml -5×10¹¹ VP/ml (specifically, 2×10⁹ VP/ml, 4×10⁹ VP/ml, 6×10⁹ VP/ml, 8×10⁹ VP/ml, 1×10¹⁰ VP/ml, 2×10¹⁰ VP/ml, 4×10¹⁰ VP/ml, 6×10¹⁰ VP/ml, 8×10¹⁰ VP/ml, 1×10¹¹ VP/ml, 2×10¹¹ VP/ml, 3×10¹¹ VP/ml, 4×10¹¹ VP/ml, 5×10¹¹ VP/ml).

Specifically, the unit dose of the above-mentioned drug is 0.05 ml -0.5 ml (such as 0.05 ml, 0.1 ml, 0.2 ml, 0.3 ml, 0.4 ml, and 0.5 ml), especially 0.05 ml -0.2 ml, and 0.1 ml.

In a fourth aspect of the present disclosure, a nebulized inhalation administration device is provided. The nebulized inhalation administration device includes the nebulization cup according to the first aspect of the present disclosure, and a nebulizer.

Specifically, the nebulizer may be an ultrasonic nebulizer, a compression nebulizer, a vibrating mesh screen nebulizer and other suitable nebulizers, especially a vibrating mesh screen nebulizer.

In a fifth aspect of the present disclosure, an application of the nebulization cup described in the first aspect in nebulized inhalation administration is provided.

Specifically, the application is application of the nebulization cup in nebulized inhalation administration of a preventive and/or therapeutic drug for a respiratory disease.

Specifically, in this application, the drug has the corresponding definition described in the third aspect of the present disclosure.

In a sixth aspect of the present disclosure, a nebulized inhalation administration method is provided, which includes a step of administering a drug to a subject through the nebulization cup described in the first aspect.

Specifically, the method is a nebulized inhalation immunization method. The drug is a vaccine, such as pneumococcal vaccine, influenza vaccine, coronavirus vaccine, chickenpox vaccine, Newcastle virus vaccine, measles vaccine, tuberculosis vaccine, dust mite allergy vaccine and the like. In some embodiments of the present disclosure, the vaccine is a coronavirus vaccine, especially SARS-CoV-2 vaccine.

Specifically, the SARS-CoV-2 vaccine may be a recombinant adenovirus vector vaccine in which the S protein gene of SARS-CoV-2 is inserted. More specifically, the recombinant adenovirus may also contain genes (full-length or partial sequences) of other structural proteins (such as M protein, E protein and N protein) of SARS-CoV-2.

Specifically, the above-mentioned adenovirus may be a human adenovirus (such as AdHu2 type, AdHu5 type and the like), an animal adenovirus vector such as a chimpanzee adenovirus vector (such as AdC6 type, AdC7 type, AdC36 type, AdC68 type and the like). In some embodiments of the present disclosure, the above-mentioned adenovirus is AdHu5.

Specifically, the content of recombinant adenovirus in the above-mentioned recombinant adenovirus vector vaccine is 1×10⁹ VP/ml -5×10¹¹ VP/ml (specifically, 2×10⁹ VP/ml, 4×10⁹ VP/ml, 6×10⁹ VP/ml, 8×10⁹ VP/ml, 1×10¹⁰ VP/ml, 2×10¹⁰ VP/ml, 4×10¹⁰ VP/ml, 6×10¹⁰ VP/ml, 8×10¹⁰ VP/ml, 1×10¹¹ VP/ml, 2×10¹¹ VP/ml, 3×10¹¹ VP/ml, 4×10¹¹ VP/ml, 5×10¹¹ VP/ml).

Specifically, the unit dose of the above-mentioned vaccine is 0.05 ml -0.5 ml (for example, 0.05 ml, 0.1 ml, 0.2 ml, 0.3 ml, 0.4 ml, and 0.5ml), especially 0.05 ml -0.2 ml, and 0.1ml.

Specifically, the method includes the following steps:
(1) the drug is nebulized by a nebulizer;
(2) the drug mist generated in step (1) is collected through the nebulization cup; and
(3) the drug mist is inhaled by a subject through the suction nozzle of the nebulization cup.

Specifically, step (3) is performed within 30 seconds (s) after the completion of mist collection (for example, 20s, 15s, 10s and 5s), especially within 10s after completion of mist collection.

Specifically, the nebulizer in step (1) may be an ultrasonic nebulizer, a compression nebulizer, a vibrating mesh screen nebulizer and other suitable nebulizers, especially a vibrating mesh screen nebulizer.

Specifically, the nebulization cup is disposable to avoid cross-infection.

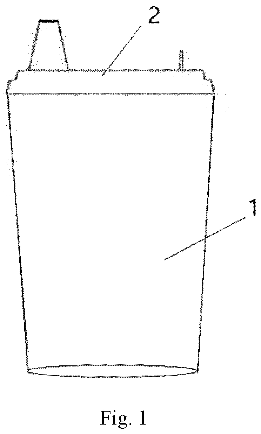

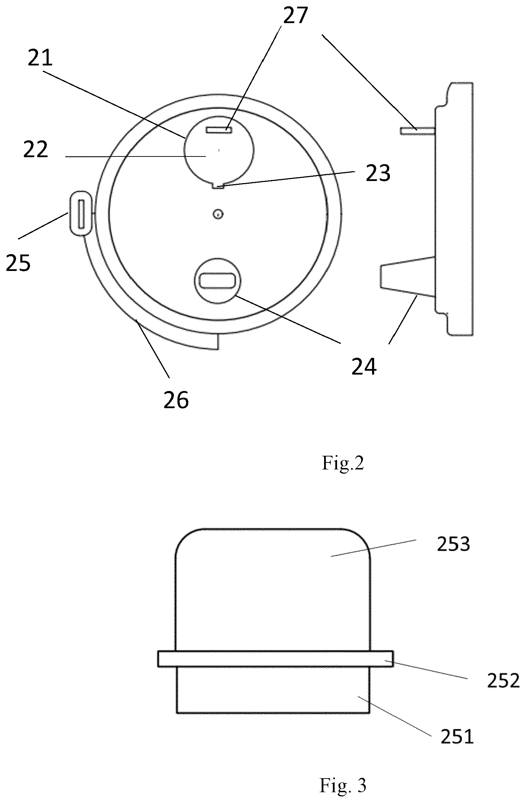

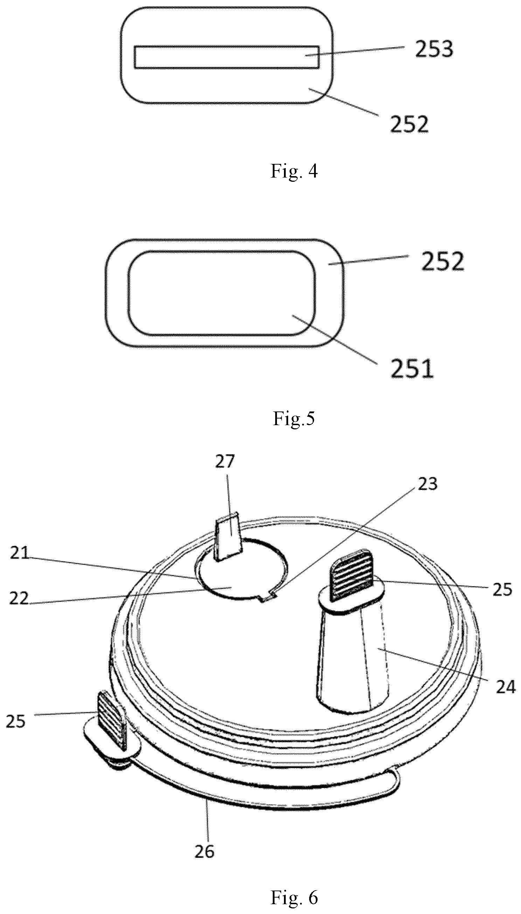

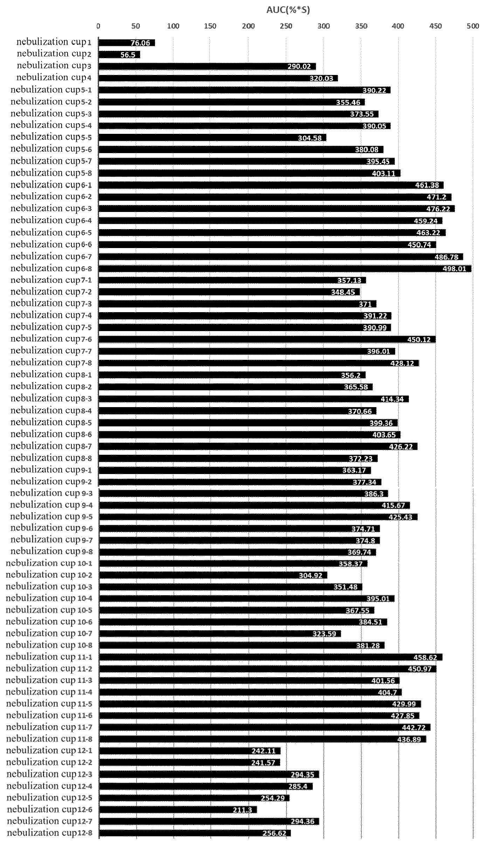

As shown in Figures 1-6, the nebulizer cup comprises a cup body 1 and a cup lid 2. The cup lid 2 includes a nebulization port 21, a flip baffle 22, a connecting part 23, a suction nozzle 24, a plug 25, a positioning line 26 and a pull tab 27. By holding the pull tab 27, the flip baffle 22 is opened, and the nebulizer is connected to the cup through the nebulization port 21. When the medication is nebulized, the mist enters the cup through the nebulization port 21, and the subject can inhale the mist through the suction nozzle 24 to achieve vaccination. After the vaccination is completed, by holding the plug 25, the pull tab 26 is broken, and then the plug 25 is inserted into the mouthpiece 24 to seal it. Additionally, by pressing down the flip flap 22 to be flush with the surface of the cup cover, the nebulization port 21 is sealed. Sealing the suction nozzle 24 and the nebulization port 21 ensures that the residual mist in the cup will not overflow into the air, protecting the environment from contamination by biological products.

Furthermore, the suction nozzle 24 is located near the edge at the top of the cup lid 2 and protrudes from the surface of the cup lid 2.

Furthermore, the cup lid 2 also includes a plug 25, the lower end of which has a plug-in part 251. The outer contour of the plug-in part 251 matches the inner wall contour of the suction nozzle 24, allowing the plug 25 to be inserted into the suction nozzle 24 and to seal it.

Furthermore, the plug 25 has a positioning part 252 extending outward from the plug-in part 251 perpendicularly, and the contour size of the positioning part 252 is larger than that of the plug-in part 251.

Furthermore, the plug 25 has a hand-held part 253 at the top end of the positioning part, which allows for manual insertion and removal of the plug 25.

Furthermore, the plug 25 is located at the edge of the cup lid 2 and is fixed on the cup lid 2 by two positioning lines 26.

Furthermore, there is a rectangular pull tab 27 adjacent to the edge of the flip flap 22, perpendicular to the flip flap 22.

Furthermore, both the nebulization port 21 and the suction nozzle 24 located at the top of the cup lid 2 are near the edge and on both sides of the central axis. The suction nozzle 24 at the top of the cup lid 2 is oval-shaped.The nebulization cup provided by the present disclosure may be used for nebulized inhalation administration of a preventive and/or therapeutic drug for a respiratory disease. Nebulization therapy mainly refers to aerosol inhalation therapy. Aerosol refers to tiny solid or liquid particles suspended in the air. Therefore, the nebulized inhalation therapy is to use the nebulization device to disperse the drug into tiny droplets or particles, which are suspended in the gas and enter the respiratory tract and lungs. The deposition of mist particles will affect the properties of mist particles. The physical mechanisms of mist deposition include impact deposition, gravity deposition, dispersion deposition, electrostatic attraction deposition and interception deposition. In order to eliminate the effect of deposition caused by electrostatic attraction on the mist particles, previous research have found that adding a food-grade antistatic agent such as edible vegetable oil raw material to the pharmaceutical preparations will cause the volume of the nebulized particles to be greater than 20 microns, which could not be delivered. The nebulization cup is treated with a static elimination device (such as ion wind wand, ion wind gun, ion fan and the like) before the nebulization cup is used. The effect of eliminating static electricity is definite. Actually, although the effect on the deposition of preparation mist particles has been improved to some extent, the effect is relatively limited.

According to the present disclosure, the antistatic agent is added to the material of the nebulization cup, and the combination of transparent plastic material and antistatic agent can keep the water droplets stable and achieve an ideal nebulization effect. The reason may be that the principle of antistatic is to form a conductive layer on the surface of the material, thereby reducing its surface resistivity and making the generated static charge leak quickly. In addition to the antistatic effect, adding the antistatic agent directly to the transparent plastic material may improve the smoothness of the plastic material surface and reduce the interception and deposition caused by the friction between the mist particles and the plastic material surface. As the antistatic agent does not play a simple antistatic or smoothing role, the present technology chooses the amount of mist, inhalable amount of mist, residual rate of liquid drug and aerodynamics of nebulized aerosol as comprehensive indexes to research the specific antistatic agent dosage of the nebulization cup, and it is found that the dosage of antistatic agent in 0.03%-10% (mass percentage) can meet the use needs, and the comprehensive indexes are better when the dosage of antistatic agent is 0.05%-5% (mass percentage). Specifically, when no antistatic agent is added, the transparent plastic nebulization cup has poor mist-carrying capacity, and the residual rate of liquid drug is high, which is almost unusable. When the dosage is 0.03%, the anti-static effect and the comprehensive performance of mist inhalation decrease. However, when the dosage is increased to 6% or 10%, the antistatic property will increase according to the dose-effect relationship, but the comprehensive performance of mist inhalation decreases. Therefore, adding a certain amount of antistatic agent can effectively maintain the stability of drug mist within a certain period of time (for example, within 20s). The particle size is stable, the drug residue in the cup is less, and the effective inhalable amount and deposition rate are ensured. The structural design of the nebulization cup further improves the inhalation performance, especially when it is used in the field of vaccines. The administration operation is simple and convenient, the inoculation efficiency can be obviously improved, and it can be used for large-scale inoculation.

### Brief Description of the Drawings

Fig. 1 is a schematic structural diagram of a nebulization cup of the present disclosure, in which a cup body 1 and a cup lid 2 are shown;
Fig. 2 is a schematic structural diagram of the cup lid of the nebulization cup according to the present disclosure, in which the left side is a top view and the right side is a cross-sectional view, and a nebulization port 21, a flip baffle 22, a connecting part 23 between the flip baffle and the cup lid, a suction nozzle 24, a plug 25, a positioning line 26 and a pull tab 27 are shown;
Fig. 3 is a schematic plan view of the plug of Fig. 2, in which a plug-in part 251, a positioning part 252 and a hand-held part 253 are shown;
Fig. 4 is a schematic top view of the plug of Fig. 2, in which the positioning part 252 and the hand-held part 253 are shown;
Fig. 5 is a schematic bottom view of the plug of Fig. 2, in which the plug-in part 251 and the positioning part 252 are shown; and
Fig. 6 is a schematic diagram of the three-dimensional structure of the nebulization cup lid of the present disclosure.
Fig. 7 shows the detection results of the inhalable amount of mist after the nebulization cups with different materials and different treatments are carrying mist for 20 seconds.

### Detailed Description

Unless otherwise defined, all scientific and technical terms used in the present disclosure have the same meanings as commonly understood by those skilled in the art related to the present disclosure.

In the present disclosure, a "subject" refers to a human who is subjected to the administration method according to the present disclosure (especially the nebulized inhalation immunization method).

The technical solutions of the present technology will be clearly and completely described below in conjunction with the embodiments of the present disclosure.

### Embodiment 1: Effect of Antistatic Agents on nebulization cups Carrying Mist

### 1. Experimental Purpose:

To research the effect of nebulizer cups carrying mist when different antistatic agents are added.

### 2. Experimental Design

Nebulization model drug: CanSino Recombinant Novel Coronavirus Vaccine (Adenovirus Type 5 Vector) liquid preparation
Nebulization volume: 0.1ml.
Antistatic Agent A: Fatty alcohol ethoxylate (anionic antistatic agent)
Antistatic Agent B: Sodium sec-alkanesulfonate (anionic antistatic agent)
Antistatic Agent C: Polyethylene glycol ester (nonionic antistatic agent)
Antistatic Agent D: Fatty acid alkyl amide (nonionic antistatic agent)
Antistatic Agent E: Polyethylene oxide (polymer antistatic agent)
Antistatic Agent F: Sodium polystyrene sulfonate (polymer antistatic agent)
Antistatic Agent G: Alkyl dicarboxymethyl ammonium succinimide (amphoteric antistatic agent)
Antistatic Agent H: Dodecyl dimethyl betaine (amphoteric antistatic agent)
Nebulization cups treated by different methods include:
   Nebulization cup 1: Nebulization cup (PP) and cup cover (PP);
   Nebulization cup 2: Nebulization cup (PP + 0.1% Antistatic Agent A) and cup cover (PP + 0.1% Antistatic Agent A);
   Nebulization cup 3: Nebulization cup (PP + 0.1% Antistatic Agent B) and cup cover (PP + 0.1% Antistatic Agent B);
   Nebulization cup 4: Nebulization cup (PP + 0.1% Antistatic Agent C) and cup cover (PP + 0.1% Antistatic Agent C);
   Nebulization cup 5: Nebulization cup (PP + 0.1% Antistatic Agent D) and cup cover (PP + 0.1% Antistatic Agent D);
   Nebulization cup 6: Nebulization cup (PP + 0.1% Antistatic Agent E) and cup cover (PP + 0.1% Antistatic Agent E);
   Nebulization cup 7: Nebulization cup (PP + 0.1% Antistatic Agent F) and cup cover (PP + 0.1% Antistatic Agent F);
   Nebulization cup 8: Nebulization cup (PP + 0.1% Antistatic Agent G) and cup cover (PP + 0.1% Antistatic Agent G);
   Nebulization cup 9: Nebulization cup (PP + 0.1% Antistatic Agent H) and cup cover (PP + 0.1% Antistatic Agent H);
   Detection/Inhalation Method: Detection 20 seconds after nebulization.
   Number of repetitions per group: 3 times.

### 3. Detection Conditions

Detection equipment: Analytical balance with a precision of one part in ten thousand.

### 4. Experimental Results and Analysis

When nebulizing 0.1ml of diluted recombinant novel coronavirus vaccine, using a PP material nebulizer cup without any treatment, the mist rapidly condensed on the walls of the cup, and only a small amount of mist remained in the cup 20 seconds after nebulization. After adding 0.1% anionic, nonionic, or polymeric antistatic agents to the nebulizer cup, both the antistatic effect and the amount of mist were improved.

### Residual amount in the cup body + cup cover after nebulization

Before the experiment, the weight of the cup body + cup cover was measured using an analytical balance with a precision of one part in ten thousand. After the nebulization ended and the mist was inhaled, the weight was measured again using the same balance: 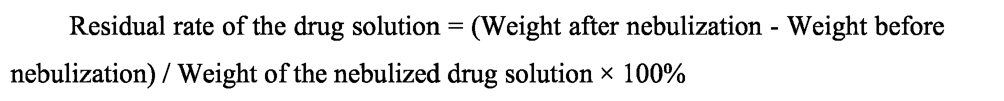 Residual rate of the drug solution = (Weight after nebulization - Weight before nebulization) / Weight of the nebulized drug solution × 100%

The results are shown in the table below:

**Table 1: The Influence of Antistatic Agents on the Residual Amount of Carried Mist**

| Antistatic | None | Anionic | | Nonionic | | Polymeric | | Amphoteric | |
|---|---|---|---|---|---|---|---|---|---|
| | | A | B | C | D | E | F | G | H |
| | Nebulization cup 1 | Nebulization cup 2 | Nebulization cup 3 | Nebulization cup 4 | Nebulization cup 5 | Nebulization cup 6 | Nebulization cup 7 | Nebulization cup 8 | Nebulization cup 9 |
| Residual Amount | 71.71% | 18.25% | 20.10% | 19.31% | 28.15% | 24.46% | 23.25% | 36.6% | 29.01% |

### Results and Analysis:

When nebulizing with a nebulization cup that has not been treated in any way, a large amount of mist remains inside the cup. The performance of nebulization cups carrying mist is significantly improved when amphoteric antistatic agents are added. Meanwhile, the performance of nebulization cups carrying mist is markedly enhanced when anionic, nonionic, or polymer antistatic agents are added. **Embodiment 2: Effect of electrostatic elimination on nebulization cups Carrying Mist**

### 1. Experimental Objective:

To research the effect of nebulization cups treated by different methods on carrying mist.

### 2. Experimental Design

Nebulization model drug: CanSino Recombinant Novel Coronavirus Vaccine (Adenovirus Type 5 Vector) liquid preparation
Nebulization volume: 0.1ml.
Antistatic Agent A is polymeric: Polyethylene glycol fatty acid ester
Antistatic Agent B is nonionic: Polyoxyethylene sorbitan monolaurate
Antistatic Agent C is anionic: Potassium cetyl phosphate
Antistatic Agent D is amphoteric: Dodecyl dimethyl glycine inner salt
Nebulization cups treated by different methods include:
   Nebulization cup 1: Nebulization cup (PP) and cup cover (PP);
   Nebulization cup 2: Nebulization cup (PLA) and cup cover (PLA);
   Nebulization cup 3: Nebulization cup (PP) and cup cover (PP), static electricity removed by ion air gun;
   Nebulization cup 4: Nebulization cup (PLA) and cup cover (PLA), static electricity removed by ion air gun;
   Nebulization cup 5-1: Nebulization cup (PP + 0.03% Antistatic Agent A) and cup cover (PP + 0.03% Antistatic Agent A);
   Nebulization cup 5-2: Nebulization cup (PLA + 0.03% Antistatic Agent A) and cup cover (PLA + 0.03% Antistatic Agent A);
   Nebulization cup 5-3: Nebulization cup (PP with 0.03% Antistatic Agent B) and cup cover (PP with 0.03% Antistatic Agent B);
   Nebulization cup 5-4: Nebulization cup (PLA with 0.03% Antistatic Agent B) and cup cover (PLA with 0.03% Antistatic Agent B);
   Nebulization cup 6-1: Nebulization cup (PP with 0.05% Antistatic Agent A) and cup cover (PP with 0.05% Antistatic Agent A);
   Nebulization cup 6-2: Nebulization cup (PLA with 0.05% Antistatic Agent A) and cup cover (PLA with 0.05% Antistatic Agent A);
   Nebulization cup 6-3: Nebulization cup (PP with 0.05% Antistatic Agent B) and cup cover (PP with 0.05% Antistatic Agent B);
   Nebulization cup 6-4: Nebulization cup (PLA with 0.05% Antistatic Agent B) and cup cover (PLA with 0.05% Antistatic Agent B);
   Nebulization cup 7-1: Nebulization cup (PP with 0.5% Antistatic Agent A) and cup cover (PP with 0.5% Antistatic Agent A);
   Nebulization cup 7-2: Nebulization cup (PLA with 0.5% Antistatic Agent A) and cup cover (PLA with 0.5% Antistatic Agent A);
   Nebulization cup 7-3: Nebulization cup (PP with 0.5% Antistatic Agent B) and cup cover (PP with 0.5% Antistatic Agent B);
   Nebulization cup 7-4: Nebulization cup (PLA with 0.5% Antistatic Agent B) and cup cover (PLA with 0.5% Antistatic Agent B);
   Nebulization cup 8-1: Nebulization cup (PP with 5% Antistatic Agent A) and cup cover (PP with 5% Antistatic Agent A);
   Nebulization cup 8-2: Nebulization cup (PLA with 5% Antistatic Agent A) and cup cover (PLA with 5% Antistatic Agent A);
   Nebulization cup 8-3: Nebulization cup (PP with 5% Antistatic Agent B) and cup cover (PP with 5% Antistatic Agent B);
   Nebulization cup 8-4: Nebulization cup (PLA with 5% Antistatic Agent B) and cup cover (PLA with 5% Antistatic Agent B);
   Nebulization cup 9-1: Nebulization cup (PP with 6% Antistatic Agent A) and cup cover (PP with 6% Antistatic Agent A);
   Nebulization cup 9-2: Nebulization cup (PLA with 6% Antistatic Agent A) and cup cover (PLA with 6% Antistatic Agent A);
   Nebulization cup 9-3: Nebulization cup (PP with 6% Antistatic Agent B) and cup cover (PP with 6% Antistatic Agent B);
   Nebulization cup 9-4: Nebulization cup (PLA with 6% Antistatic Agent B) and cup cover (PLA with 6% Antistatic Agent B);
   Nebulization cup 10-1: Nebulization cup (PP with 10% Antistatic Agent A) and cup cover (PP with 10% Antistatic Agent A);
   Nebulization cup 10-2: Nebulization cup (PLA with 10% Antistatic Agent A) and cup cover (PLA with 10% Antistatic Agent A);
   Nebulization cup 10-3: Nebulization cup (PP with 10% Antistatic Agent B) and cup cover (PP with 10% Antistatic Agent B);
   Nebulization cup 10-4: Nebulization cup (PLA with 10% Antistatic Agent B) and cup cover (PLA with 10% Antistatic Agent B).

### 3. Detection Conditions

Detection equipment: SYMPATEC company HELOS & INHALER inhalation aerosol, powder aerosol test laser particle size analyzer.

Detection/inhalation method: Detection 20 seconds after nebulization.

Number of repetitions per group: 3 times.

Detection indicators: The duration of the optical concentration Copt (Optical Concentration/%) ≥ 20% interval and the optical concentration at the corresponding time points.

### 4. Experimental Results and Analysis

Calculate the area under the mist optical concentration-time curve, referred to as "AUC" (%*S), to represent the amount of mist, and use the amount of mist as an indicator to study the material of the Nebulization cup. The results are shown in Figure 7. Results and analysis:
When nebulizing 0.1ml of diluted recombinant novel coronavirus vaccine using a Nebulization cup that has not been treated, the mist rapidly condenses on the cup wall, and only a small amount of mist remains in the cup 20 seconds after nebulization. After treating the Nebulization cup with an ion air gun to remove static electricity for carrying mist, the results show an increase in inhalable volume 20 seconds after nebulization, but the effect is still not satisfactory. After adding 0.03% antistatic agent to the Nebulization cup, both the antistatic effect and the amount of mist are improved; according to the present invention, after adding 0.05% or 0.5% antistatic agent to the Nebulization cup, the performance of the cup in carrying mist is further improved, and its effect is significantly better than that after static electricity removal by the ion air gun. When the addition amount is 6% or 10%, according to the dose-effect relationship, the antistatic property will increase, but the amount of mist decreases.

### Residual amount in cup body + cup lid after nebulization

Before the experiment, a one-ten-thousand balance is used to weigh the cup body + cup lid. After the nebulization is completed, a one-ten-thousand balance is used to weigh again after the mist is inhaled: 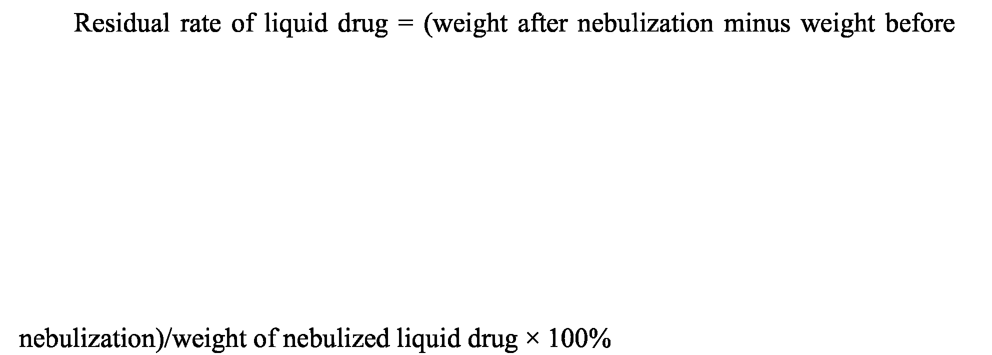 Residual rate of liquid drug = (weight after nebulization minus weight before nebulization)/weight of nebulized liquid drug × 100%

The results are shown in the table below.

**Table 2 Residual rate of liquid drug in different nebulization cups**

| nebulization cup | AUC(%*s) |
|---|---|
| nebulization cup 1 | 76.06 |
| nebulization cup 2 | 56.5 |
| nebulization cup 3 | 290.02 |
| nebulization cup 4 | 320.03 |
| nebulization cup 5-1 | 390.22 |
| nebulization cup 5-2 | 355.46 |
| nebulization cup 5-3 | 373.55 |
| nebulization cup 5-4 | 390.05 |
| nebulization cup 5-5 | 304.58 |
| nebulization cup 5-6 | 380.08 |
| nebulization cup 5-7 | 395.45 |
| nebulization cup 5-8 | 403.11 |
| nebulization cup 6-1 | 461.38 |
| nebulization cup 6-2 | 471.2 |
| nebulization cup 6-3 | 476.22 |
| nebulization cup 6-4 | 459.24 |
| nebulization cup 6-5 | 463.22 |
| nebulization cup 6-6 | 450.74 |
| nebulization cup 6-7 | 486.78 |
| nebulization cup 6-8 | 498.01 |
| nebulization cup 7-1 | 357.13 |
| nebulization cup 7-2 | 348.45 |
| nebulization cup 7-3 | 371.00 |
| nebulization cup 7-4 | 391.22 |
| nebulization cup 7-5 | 390.99 |
| nebulization cup 7-6 | 450.12 |
| nebulization cup 7-7 | 396.01 |
| nebulization cup 7-8 | 428.12 |
| nebulization cup 8-1 | 356.2 |
| nebulization cup 8-2 | 365.58 |
| nebulization cup 8-3 | 414.34 |
| nebulization cup 8-4 | 370.66 |
| nebulization cup 8-5 | 399.36 |
| nebulization cup 8-6 | 403.65 |
| nebulization cup 8-7 | 426.22 |
| nebulization cup 8-8 | 372.23 |
| nebulization cup 9-1 | 363.17 |
| nebulization cup 9-2 | 377.34 |
| nebulization cup 9-3 | 386.3 |
| nebulization cup 9-4 | 415.67 |
| nebulization cup 9-5 | 425.43 |
| nebulization cup 9-6 | 374.71 |
| nebulization cup 9-7 | 374.80 |
| nebulization cup 9-8 | 369.74 |
| nebulization cup 10-1 | 358.37 |
| nebulization cup 10-2 | 304.92 |
| nebulization cup 10-3 | 351.48 |
| nebulization cup 10-4 | 395.01 |
| nebulization cup 10-5 | 367.55 |
| nebulization cup 10-6 | 384.51 |
| nebulization cup 10-7 | 323.59 |
| nebulization cup 10-8 | 381.28 |
| nebulization cup 11-1 | 458.62 |
| nebulization cup 11-2 | 450.97 |
| nebulization cup 11-3 | 401.56 |
| nebulization cup 11-4 | 404.70 |
| nebulization cup 11-5 | 429.99 |
| nebulization cup 11-6 | 427.85 |
| nebulization cup 11-7 | 442.72 |
| nebulization cup 11-8 | 436.89 |
| nebulization cup 12-1 | 242.11 |
| nebulization cup 12-2 | 241.57 |
| nebulization cup 12-3 | 294.35 |
| nebulization cup 12-4 | 285.40 |
| nebulization cup 12-5 | 254.29 |
| nebulization cup 12-6 | 211.3 |
| nebulization cup 12-7 | 294.36 |
| nebulization cup 12-8 | 256.62 |

**Table 3 The average residue rate in different nebulization cups**

| nebulization cup | antistatic agent | 0% | static removal by an ionized air | 0.03% | 0.05% | 0.5% | 5% | 6% | 10% | 11% | 12% |
|---|---|---|---|---|---|---|---|---|---|---|---|
| PP | A | 71.71% | 24.06% | 25.25% | 11.82% | 9.52% | 10.52% | 18.36% | 21.39% | 29.55% | 43.90% |
| | B | | | 28.22% | 13.16% | 10.41% | 13.11% | 22.32% | 25.46% | 26.63% | 47.98% |
| | C | | | 10.92% | 11.56% | 11.23% | 20.51% | 28.99% | 11.55% | 14.04% | 38.44% |
| | D | | | 16.27% | 18.22% | 12.44% | 16.87% | 26.45% | 23.31% | 20.17% | 38.26% |
| PLA | A | 68.25% | 18.71% | 34.44% | 12.45% | 8.75% | 14.58% | 19.87% | 28.87% | 10.71% | 58.41% |
| | B | | | 32.95% | 13.94% | 9.67% | 16.01% | 30.24% | 32.29% | 21.40% | 55.72% |
| | C | | | 32.80% | 12.32% | 16.59% | 27.99% | 11.14% | 20.27% | 21.71% | 50.21% |
| | D | | | 38.36% | 15.33% | 10.23% | 28.10% | 15.75% | 24.79% | 27.28% | 46.61% |

### Results and Analysis:

As the content of the antistatic agent changes, the residue of the mist in the nebulization cup is also continuously changing. When the content of the antistatic agent is between 0.03% and 10%, the residue of the mist in the nebulization cup is minimal, and the results are superior to or essentially the same as those of the nebulization cups treated with static removal by an ionized air gun. When the content of the antistatic agent is between 0.05% and 5%, the effect of preventing mist residue in different nebulization cups reaches its optimum.

### Embodiment 3: Effect of inhalation with a nebulization cup on the particle size of vaccine particles

### 1. Experimental purpose

To research the effect of anti-static nebulization cup on mist carrying by nebulization cups.

### 2. Experimental design

Nebulization model drug: CanSino Recombinant Novel Coronavirus Vaccine (Adenovirus Type 5 Vector) liquid preparation
Nebulization amount: 0.1 ml.
Nebulization cups: Nebulization cups 6-1, 6-3, 7-1, 7-3, 8-1 and 8-3 in Embodiment 1 are selected for research.

### 3. Detection conditions

Detection device: Inhalation preparation particle size detector.
Detection conditions: 10s after nebulization.
Detection items: proportion of particles with particle sizes (X_{10.3}, X_{50.3}, X_{90.3}) smaller than 5.25 microns.

### 4. Experimental results and analysis

The aerodynamic distribution of aerosol nebulized by the nebulization cup is detected by the inhalation preparation particle size detector, and the detection is carried out three times in parallel, as shown in the table below.

**Table 4 Aerodynamic distribution results of nebulized aerosol**

| Serial number | X_{10.3}(µm) | X_{50.3}(µm) | X_{90.3}(µm) | Proportion of particles (%) with particle sizes < 5.25 µm |
|---|---|---|---|---|
| 6-1 | 1.06 | 2.85 | 8.39 | 78.13 |
| 6-2 | 1.02 | 2.88 | 8.62 | 76.78 |
| 7-1 | 0.99 | 2.88 | 8.32 | 77.80 |
| 7-2 | 0.71 | 2.43 | 6.76 | 82.82 |
| 8-1 | 0.76 | 2.51 | 7.15 | 81.56 |
| 8-2 | 0.66 | 2.41 | 6.64 | 83.98 |

### Results and analysis:

The PP nebulization cup is used, and the content of antistatic agent is 0.05%-5%. The aerodynamic particle size of the detected mist is similar to that of the particle size less than 5.25 microns with no significant difference, and it meets the requirements of nebulized inhalation administration.

### Embodiment 4: Comparison of the inhalable amount between the inhalation method using the anti-static nebulization cup and the inhalation method using the commercially available suction nozzle

### 1. Experimental purpose

Traditional nebulized inhalation administration (that is, inhalation through suction nozzle) adopts nebulizing while inhaling, which brings drugs to the lungs. If it is replaced by nebulization cup inhalation, it is necessary to collect the mist in the nebulization cup and then inhale it in one breath. In this embodiment, the amount of drug in the way of one-breath inhalation after using the antistatic nebulization cup to carry the mist is researched, and the comparison is made with the way of inhaling through a suction nozzle.

### 2. Experimental design

Nebulization model drug: CanSino Recombinant Novel Coronavirus Vaccine (Adenovirus Type 5 Vector) liquid preparation
Nebulization amount: 0.1 ml.
Nebulization cup: The nebulization cup 6-1 in Embodiment 1 is selected for research.

### 3. Detection conditions

Suction nozzle inhalation mode: the adult breathing mode of the respiration simulator, which collects the inhalable drug to the filter membrane.

Nebulization cup suction mode: the suction speed of the pump is adjusted to 15 L/min, and the filter fixing device is connected to the pump. 10s after the end of the nebulization, the cup mouth is connected to the filter membrane connection port, and the mist is collected to the filter membrane.

### 4. Experimental results and analysis

Each mode is repeated 6 times. After that, the vaccine on the filter is eluted, and the virus particles are quantified by ELISA method. The results are shown in the following table.

**Table 5 Detection results of inhalable amount**

| Inhalation device | Inhalable amount | Inhalable amount (mean) |
|---|---|---|
| Inhale using a suction nozzle | 48% | 53.50% |
| | 59% | |
| | 53% | |
| | 58% | |
| | 52% | |
| | 51% | |
| Inhalation using a nebulization cup | 52% | 48.30% |
| | 46% | |
| | 50% | |
| | 49% | |
| | 47% | |
| | 46% | |

### Results and analysis:

The results show that the inhalable amount when using the nebulization cup for administration is 48.3%, which is not significantly different from the inhalable amount when using the suction nozzle. The results show that the nebulization cup may be used to replace the suction nozzle.

The foregoing descriptions are only preferred embodiments of the present disclosure, and are not intended to limit the present invention.

The present invention is defined by the appended claims.

## Claims

1. A nebulization cup, wherein the nebulization cup comprises a cup body (1) and a cup lid (2), a material of the cup body is transparent plastic, **characterized in that** the cup body comprising an antistatic agent of 0.05%-0.5% in terms of mass percentage; the nebulization cup is applicated in nebulized inhalation administration of a vaccine.

2. The nebulization cup according to claim 1, **characterized in that** the material of the cup body is selected from a group consisting of polypropylene (PP), polylactic acid (PLA), polyethylene (PE), polyvinyl chloride (PVC), polystyrene (PS) and polycarbonate (PC).

3. The nebulization cup according to claim 1, **characterized in that** a material of the cup lid is plastic, the plastic is selected from a group consisting of polypropylene (PP), polylactic acid (PLA), polyethylene (PE), polyvinyl chloride (PVC), polystyrene (PS) and polycarbonate (PC).

4. The nebulization cup according to claim 1, **characterized in that** the cup lid comprising an antistatic agent of 0.05%-0.5% in terms of mass percentage.

5. The nebulization cup according to claim 1, **characterized in that** the cup lid is provided with a mist inlet (21) and a suction nozzle (24).

6. The nebulization cup according to claim 5, **characterized in that** the mist inlet is provided with a closing member (22); a suction port of the suction nozzle is provided with a closing member (25).

7. The nebulization cup according to any one of claims 1-6, **characterized in that** the antistatic agent is one of or a combination of more than one of anionic antistatic agent, zwitterionic antistatic agent, nonionic antistatic agent and polymer antistatic agent.

8. The nebulization cup according to claim 7, **characterized in that** the anionic antistatic agent is one of or a combination of more than one of alkyl sulfonate, alkyl phosphate, copolymer salt of maleic anhydride and other monomer copolymer salts, polyacrylates and polystyrene sulfonates; the zwitterionic antistatic agent is amphoteric alkyl imidazoline salt and alkyl amino acid; the nonionic antistatic agent is one of or a combination of more than one of polyhydroxy alcohol fatty acid ester and polyoxyethylene additive; the polymer antistatic agent is one of or a combination of more than one of polyoxyethylene fatty ether, polyoxyethylene alkylbenzene ether, polyethylene glycol fatty acid ester and polyacrylic acid derivative.

9. The nebulization cup according to claim 7, **characterized in that** the nonionic antistatic agent mentioned is selected from one of or a combination of more than one of fatty alcohol ethoxylates, alkyl phenol ethoxylates, and mono- and di-glycerides of fatty acids; the amphoteric antistatic agent is alkyl dicarboxymethyl ammonium succinimide and/or dodecyl dimethyl betaine; the polymer antistatic agent is selected from one of or a combination of more than one of ethylene oxide-propylene oxide adducts of ethylenediamine, poly(4-vinylpyridine) type polymeric soaps, octylphenyl styrenes, and styrene sulfonate copolymeric soaps.

10. The nebulization cup according to claim 1, **characterized in that** the nebulization cup has a volume of 300-800 ml.

11. The nebulization cup according to claim 10, **characterized in that** the nebulization cup has a volume of 500 ml.

12. A method for preparing the nebulization cup according to any one of claims 1-11, the method comprising a step of adding an antistatic agent component or an antistatic masterbatch to a raw material for preparing the cup body, **characterized by** the cup body comprising an antistatic agent of 0.05%-0.5% in terms of mass percentage.

13. A application of the nebulization cup according to any one of claims 1-11 in preparation of a device for nebulized inhalation administration, wherein the nebulization cup comprises a cup body and a cup lid, a material of the cup body is transparent plastic, **characterized by** the cup body comprising an antistatic agent of 0.05%-0.5% in terms of mass percentage.

14. The application according to claim 13, **characterized in that** the application is application of the nebulization cup in the preparation of a device for nebulized inhalation administration of a vaccine for a respiratory disease.

## Patentansprüche

1. Vernebelungsbecher, wobei der Vernebelungsbecher einen Becherkörper (1) und einen Becherdeckel (2) umfasst, ein Material des Becherkörpers transparenter Kunststoff ist, **dadurch gekennzeichnet, dass** der Becherkörper ein Antistatikum von 0,05 %- 0,5 % in Bezug auf den Massenprozentsatz umfasst; der Verneblungsbecher bei der vernebelten Inhalationsverabreichung eines Impfstoffs angewendet wird.

2. Vernebelungsbecher nach Anspruch 1, **dadurch gekennzeichnet, dass** das Material des Becherkörpers aus einer Gruppe ausgewählt ist, die aus Polypropylen (PP), Polymilchsäure (PLA), Polyethylen (PE), Polyvinylchlorid (PVC), Polystyrol (PS) und Polycarbonat (PC) besteht.

3. Vernebelungsbecher nach Anspruch 1, **dadurch gekennzeichnet, dass** ein Material des Becherdeckels Kunststoff ist, wobei der Kunststoff aus einer Gruppe ausgewählt ist, die aus Polypropylen (PP), Polymilchsäure (PLA), Polyethylen (PE), Polyvinylchlorid (PVC), Polystyrol (PS) und Polycarbonat (PC) besteht.

4. Vernebelungsbecher nach Anspruch 1, **dadurch gekennzeichnet, dass** der Becherdeckel ein Antistatikum von 0,05 %-0,5 % in Bezug auf den Massenprozentsatz umfasst.

5. Vernebelungsbecher nach Anspruch 1, **dadurch gekennzeichnet, dass** der Becherdeckel mit einem Dunsteinlass (21) und einer Saugdüse (24) bereitgestellt ist.

6. Vernebelungsbecher nach Anspruch 5, **dadurch gekennzeichnet, dass** der Dunsteinlass mit einem Verschlusselement (22) bereitgestellt ist; eine Saugöffnung der Saugdüse mit einem Verschlusselement (25) bereitgestellt ist.

7. Vernebelungsbecher nach einem der Ansprüche 1-6, **dadurch gekennzeichnet, dass** das Antistatikum eines von oder eine Kombination von mehr als einem von anionischem Antistatikum, zwitterionischem Antistatikum, nichtionischem Antistatikum und polymerem Antistatikum ist.

8. Vernebelungsbecher nach Anspruch 7, **dadurch gekennzeichnet, dass** das anionische Antistatikum eines oder eine Kombination von mehr als einem von Alkylsulfonat, Alkylphosphat, Copolymersalz von Maleinsäureanhydrid und anderen monomeren Copolymersalzen, Polyacrylaten und Polystyrolsulfonaten ist; das zwitterionische Antistatikum ein amphoteres Alkylimidazolinsalz und eine Alkylaminosäure ist; das nichtionische Antistatikum eines oder eine Kombination von mehr als einem von Polyhydroxyalkoholfettsäureester und Polyoxyethylenadditiv ist; das polymere Antistatikum eines oder eine Kombination von mehr als einem von Polyoxyethylenfettether, Polyoxyethylenalkylbenzolether, Polyethylenglycolfettsäureester und Polyacrylsäurederivat ist.

9. Vernebelungsbecher nach Anspruch 7, **dadurch gekennzeichnet, dass** das genannte nichtionische Antistatikum aus einem von oder einer Kombination von mehr als einem von Fettalkoholethoxylaten, Alkylphenolethoxylaten und Mono- und Diglyceriden von Fettsäuren ausgewählt ist; das amphotere Antistatikum Alkyldicarboxymethylammoniumsuccinimid und/oder Dodecyldimethylbetain ist; das polymere Antistatikum aus einem von oder einer Kombination von mehr als einem von Ethylenoxid-Propylenoxid-Addukten von Ethylendiamin, polymeren Seifen vom Poly(4-vinylpyridin)-Typ, Octylphenylstyrolen und Styrolsulfonatcopolymerseifen ausgewählt ist.

10. Vernebelungsbecher nach Anspruch 1, **dadurch gekennzeichnet, dass** der Vernebelungsbecher ein Volumen von 300-800 ml aufweist.

11. Vernebelungsbecher nach Anspruch 10, **dadurch gekennzeichnet, dass** der Vernebelungsbecher ein Volumen von 500 ml aufweist.

12. Verfahren zum Herstellen des Vernebelungsbechers nach einem der Ansprüche 1-11, wobei das Verfahren einen Schritt des Hinzufügens einer Antistatikumskomponente oder eines antistatischen Masterbatches zu einem Rohmaterial zum Herstellen des Becherkörpers umfasst, **dadurch gekennzeichnet, dass** der Becherkörper ein Antistatikum von 0,05 %-0,5 % in Bezug auf den Massenprozentsatz umfasst.

13. Anwendung des Vernebelungsbechers nach einem der Ansprüche 1-11 zur Herstellung einer Vorrichtung zur vernebelten Inhalationsverabreichung, wobei der Vernebelungsbecher einen Becherkörper und einen Becherdeckel umfasst, ein Material des Becherkörpers ein transparenter Kunststoff ist, **dadurch gekennzeichnet, dass** der Becherkörper ein Antistatikum von 0,05 %-0,5 % in Bezug auf den Massenprozentsatz umfasst.

14. Anwendung nach Anspruch 13, **dadurch gekennzeichnet, dass** die Anwendung eine Anwendung des Vernebelungsbechers bei der Herstellung einer Vorrichtung zur vernebelten Inhalationsverabreichung eines Impfstoffs für eine Atemwegserkrankung ist.

## Revendications

1. Coupelle de nébulisation, dans laquelle la coupelle de nébulisation comprend un corps de coupelle (1) et un couvercle de coupelle (2), un matériau du corps de coupelle est un plastique transparent, **caractérisée en ce que** le corps de coupelle comprend un agent antistatique à raison de 0,05 % à 0,5 % en termes de pourcentage massique ; la coupelle de nébulisation est appliquée dans l'administration par inhalation par nébulisation d'un vaccin.

2. Coupelle de nébulisation selon la revendication 1, **caractérisée en ce que** le matériau du corps de coupelle est choisi dans un groupe constitué de polypropylène (PP), d'acide polylactique (PLA), de polyéthylène (PE), de polychlorure de vinyle (PVC), de polystyrène (PS) et de polycarbonate (PC).

3. Coupelle de nébulisation selon la revendication 1, **caractérisée en ce qu'**un matériau du couvercle de coupelle est un plastique, le plastique étant choisi dans un groupe constitué de polypropylène (PP), d'acide polylactique (PLA), de polyéthylène (PE), de polychlorure de vinyle (PVC), de polystyrène (PS) et de polycarbonate (PC).

4. Coupelle de nébulisation selon la revendication 1, **caractérisée en ce que** le couvercle de coupelle comprend un agent antistatique à raison de 0,05 % à 0,5 % en termes de pourcentage massique.

5. Coupelle de nébulisation selon la revendication 1, **caractérisée en ce que** le couvercle de coupelle est pourvu d'une entrée de brouillard (21) et d'un embout d'aspiration (24).

6. Coupelle de nébulisation selon la revendication 5, **caractérisée en ce que** l'entrée de brouillard est pourvue d'un élément de fermeture (22) ; un orifice d'aspiration de l'embout d'aspiration est pourvu d'un élément de fermeture (25).

7. Coupelle de nébulisation selon l'une quelconque des revendications 1 à 6, **caractérisée en ce que** l'agent antistatique est l'un parmi ou une combinaison de plus d'un parmi un agent antistatique anionique, un agent antistatique zwitterionique, un agent antistatique non ionique et un agent antistatique polymère.

8. Coupelle de nébulisation selon la revendication 7, **caractérisée en ce que** l'agent antistatique anionique est l'un parmi ou une combinaison de plus d'un parmi un sulfonate d'alkyle, un phosphate d'alkyle, un sel de copolymère d'anhydride maléique et d'autres sels de copolymères de monomères, des polyacrylates et des sulfonates de polystyrène ; l'agent antistatique zwitterionique est un sel d'imidazoline d'alkyle amphotère et un acide aminé alkylé ; l'agent antistatique non ionique est l'un parmi ou une combinaison de plus d'un parmi un ester d'acide gras d'alcool polyhydroxylé et un additif de polyoxyéthylène ; l'agent antistatique polymère est l'un parmi ou une combinaison de plus d'un parmi un éther gras de polyoxyéthylène, un éther d'alkylbenzène de polyoxyéthylène, un ester d'acide gras de polyéthylèneglycol et un dérivé d'acide polyacrylique.

9. Coupelle de nébulisation selon la revendication 7, **caractérisée en ce que** l'agent antistatique non ionique mentionné est choisi parmi l'un parmi ou une combinaison de plus d'un parmi des éthoxylates d'alcools gras, des éthoxylates d'alkylphénols et des mono- et diglycérides d'acides gras ; l'agent antistatique amphotère est le succinimide d'alkyl dicarboxyméthylammonium et/ou la dodécyl diméthyl bétaïne ; l'agent antistatique polymère est choisi parmi l'un parmi ou une combinaison de plus d'un parmi des produits d'addition d'oxyde d'éthylène-oxyde de propylène d'éthylènediamine, des savons polymères de type poly(4-vinylpyridine), des octylphénylstyrènes et des savons copolymères de styrène sulfonate.

10. Coupelle de nébulisation selon la revendication 1, **caractérisée en ce que** la coupelle de nébulisation présente un volume de 300 à 800 ml.

11. Coupelle de nébulisation selon la revendication 10, **caractérisée en ce que** la coupelle de nébulisation présente un volume de 500 ml.

12. Procédé permettant la fabrication de la coupelle de nébulisation selon l'une quelconque des revendications 1 à 11, le procédé comprenant une étape d'ajout d'un composant d'agent antistatique ou d'un mélange maître antistatique à une matière première pour la fabrication du corps de coupelle, **caractérisé en ce que** le corps de coupelle comprend un agent antistatique à raison de 0,05 % à 0,5 % en termes de pourcentage massique.

13. Application de la coupelle de nébulisation selon l'une quelconque des revendications 1 à 11 dans la fabrication d'un dispositif d'administration par inhalation par nébulisation, dans laquelle la coupelle de nébulisation comprend un corps de coupelle et un couvercle de coupelle, un matériau du corps de coupelle est un plastique transparent, **caractérisée en ce que** le corps de coupelle comprend un agent antistatique à raison de 0,05 % à 0,5 % en pourcentage massique.

14. Application selon la revendication 13, **caractérisée en ce que** l'application est une application de la coupelle de nébulisation dans la fabrication d'un dispositif pour l'administration par inhalation par nébulisation d'un vaccin destiné à une maladie respiratoire.
